# EUROPEAN PATENT SPECIFICATION

(11) **EP 1 401 342 B1**
(45) Date of publication and mention of the grant of the patent: **12.01.2005**
(21) Application number: 02730528.3
(22) Date of filing: 18.06.2002
(51) Int. Cl.: A61B 17/43

(54) **APPARATUS FOR AND METHODS OF PREPARING SPERM**
VERFAHREN UND GERÄT ZUR HERSTELLUNG VON SPERMIEN
APPAREIL ET PROCEDES DESTINES A LA PREPARATION DU SPERME

(30) Priority: 20.06.2001 GB 0115105; 03.11.2001 GB 0126593
(43) Date of publication of application: 31.03.2004
(73) Proprietor: Fertility Medical Equipment (Scotland) Limited, Motherwell ML1 3BJ (GB)
(72) Inventor: ABED, Farhang Fathemieh Hospital, Pasdaran Street Hamadan (IR)
(74) Representative: Henderson, Wendy Agnes
(86) International application number: PCT/GB2002/002793
(87) International publication number: WO 2002/102257

(56) References cited:
- US-A- 5 296 375
- US-A- 5 691 194

## Description

This invention relates generally to fertility, and more particularly to apparatus for and methods of preparing sperm for intracytoplasmic sperm injection (ICSI).

### Background of the invention

The introduction of ICSI which involves the direct injection of a sperm into an oocyte has bypassed the natural selection mechanisms for the fertilizing sperm. When performing ICSI a population of sperm are selected from the raw semen, using various methods of preparation. This population should have an increased proportion of motile sperm with normal morphology. However, it is not clear from the hundreds of sperm being observed which one is best qualified for injection.

Sperm preparation for ICSI uses the same conventional methods that are used for IVF, including "Percol" density gradient and "Swim up" methods. These methods can involve either centrifugation or the use of chemicals. It has been shown that centrifugal force generates the production of reactive oxygen species that may damage sperm and impair their fertilization potential. (R.J. Aitken and J.S. Clarkson "*Cellular basis of defecti ve sperm functi on and its association with the genesis of reactive oxygen species by human spermatozoa*," J. Reprod. Fertil. 1991;6: pages 173-176). However, "Percol" has been used largely in the setting of laboratory research and its clinical use is associated with certain disadvantages. Some batches have been found to contain high levels of endotoxin, making,them unsuitable for clinical use. In late 1996, "Percol" was withdrawn from clinical use as a sperm separation medium (Guneet Makkar, Hung-Yu Ng, et al. "*Comparison of two colloidal silica-based sperm separation media with a non-silica-based medium,"* Fertil. Steril. 1999; 72: pages 796-802.

It has been reported that when sperm are put into a fluid flow, the motile sperm rapidly align themselves and swim against the flow (F. Abed. *"The new finding of a phenomenon in sperm motility: the spermatozoa swims against flow* " *-* from "In vitro fertilization and assisted reproduction", edited by V. Gomel and P.C.K. Leung, Monduzzi Editore, 1997: pages 13-15). Non-motile and sluggish sperm, along with other cellular components, are washed downstream away from the motile sperm. Cilia have been shown to be present in endometrial cells of many mammals. Ciliary currents in both the fallopian tubes and the uterus move in the same direction and extend towards the external os. One may expect that this flow performs two functions. Firstly, this flow acts as a guide for sperm, leading sperm with the correct motility parameters towards the site of fertilization at the ampoule of the fallopian tubes. Secondly, this flow acts as a natural selection mechanism to optimize the quality of sperm able to reach the fertilization site.

In US-A-4326026 there is described a number of fractionating columns in which one establishes laminar flow. Nutrient is pumped into vertical pipettes and when the laminar flow has been established sperm is injected from a syringe. The sperm is then distributed evenly throughout the columns and samples can be withdrawn at different levels.

US-A-4759344 describes a rudimentary arrangement for separating motile sperm from semen. There is however no means of establishing a flow of fluid and nor is there any constriction which would produce a capillary flow.

US-A-5296375 describes devices and methods for clinical analysis of sperm samples. The devices have an inlet port and a flow channel which is referred to as a mesoscale flow channel and an outlet port for harvesting sperm similar to the device of claim 1.

US-A-5686302 shows a sperm separating device in which a fluid bridge is created through which the sperm can pass, but no fluid flow is established.

### Summary of the invention

It is an object of the present invention to use sperm alignment against fluid flow to separate motile, morphologically normal sperm suitable for ICSI from semen. In addition, the method and apparatus of the present invention have several advantages over conventional methods:
- the present invention utilizes the sperm alignment against flow phenomenon;
- it does not induce any damage to sperm, because the procedure does not require any centrifugation or chemicals;
- it is rapid and simple;
- the process of sperm separation is under direct observation and can be easily controlled. Other methods tend to be blind and there is little or no control while performing the process and it is not until the end of the process that the quality of sperm can be evaluated.

In accordance with one aspect of the present invention there is provided a sperm separation device comprising a tubular conduit defining an upstream zone for receiving semen and a downstream zone from which sperm are arranged to be harvested, the upstream and downstream zones being separated by constriction means within the conduit, the constriction means being arranged to establish capillary flow of a fluid medium from the upstream zone to the downstream zone, whereby motile sperm in semen inserted into the conduit upstream from the constriction means and subjected to the flow of the fluid medium are enabled, having passed through the constriction means, to maintain a position in the downstream zone for harvesting.

The conduit preferably has a relatively large upstream end and a relatively small downstream end.

Preferably, the constriction means comprises means within the conduit defining a substantially hour-glass-shaped passageway for the sperm and fluid. The constriction means is preferably provided adjacent to the downstream end of the conduit.

Also in accordance with the present invention there is provided a method of separating sperm, which comprises inserting sperm-containing semen into a tubular conduit upstream from constriction means within the conduit which is arranged to create capillary flow therethrough, filling the conduit upstream from the constriction means with fluid, thereby to cause flow of semen and fluid through the constriction means, and harvesting motile sperm from the downstream side of the constriction means.

Preferably, the population of motile sperm is aspirated from the conduit using a pipette.

Preferably, the tubular conduit is made of glass which is not reactive with the semen or fluid medium.

### Brief description of the drawings

A more detailed description of the present invention will now be given, with reference to the accompanying drawings, wherein like reference characters refer to like parts throughout the several views, and in which:
Fig. 1 is a schematic diagram of one embodiment of a sperm separation device in accordance with the invention placed on a microscope slide;
Fig. 2 is a schematic view, on an enlarged scale, of the distal end of the device shown in Fig. 1 and illustrating the use of a pipette for aspirating the motile sperm;
Fig. 3 is a schematic illustration of a second embodiment of sperm separation device in accordance with the present invention; and
Fig. 4 is a schematic view, on an enlarged scale, of the distal end of the device shown in Fig. 3 and illustrating the use of a pipette for aspirating the motile sperm.

### Detailed description of the preferred embodiment

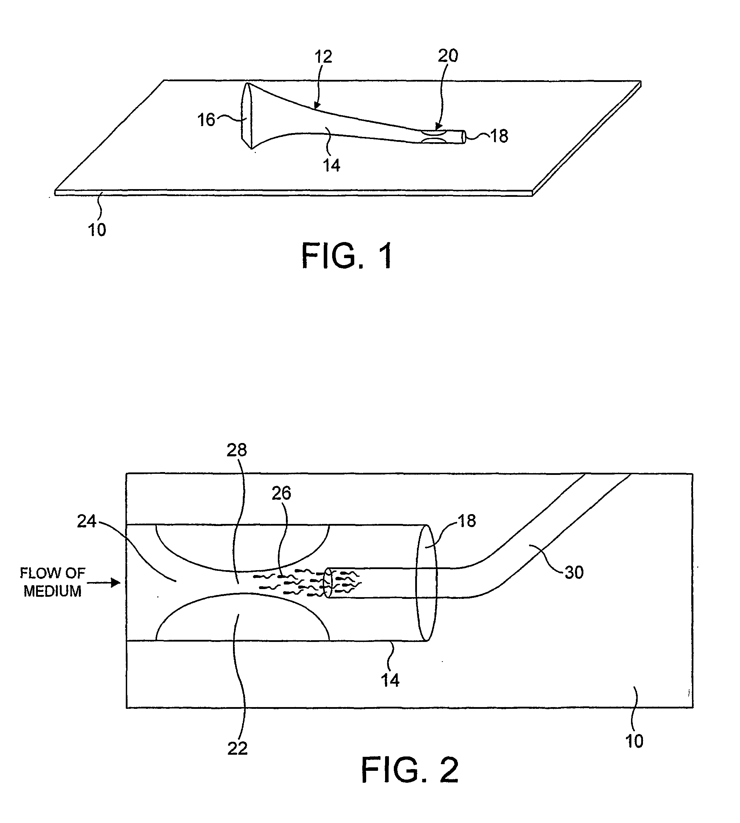

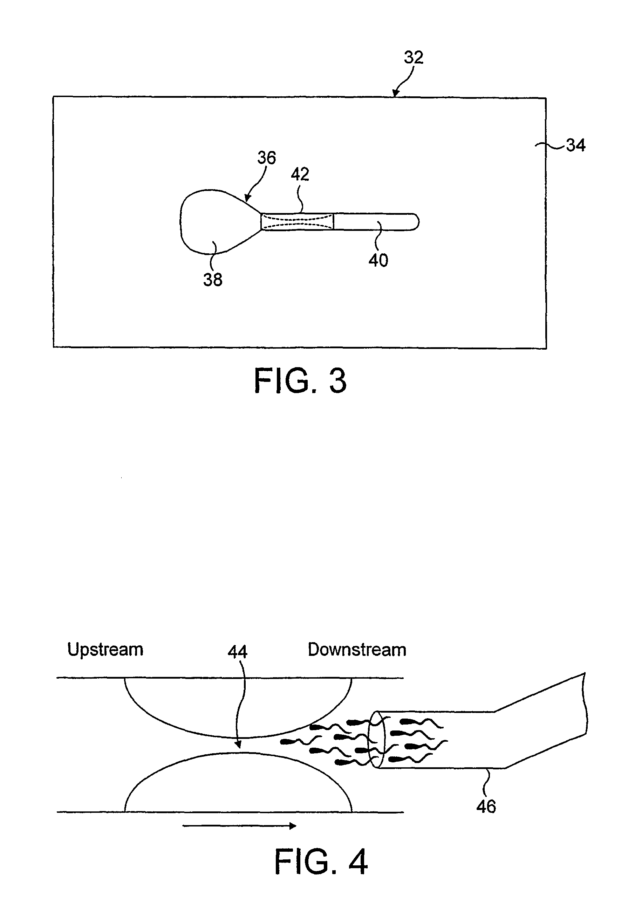

Referring first to the embodiment shown in Figs. 1 and 2, Fig. 1 shows a microscope slide 10 on which is positioned a sperm separation device 12 in accordance with the invention. The device comprises a glass tube 14 which has a relatively large inlet end 16 and a relatively small outlet or distal end 18. Within the tube 14, adjacent to the distal end 18, there is provided a constriction 20. The tube 14 is funnel-shaped from the inlet 16 to the constriction 20 and is cylindrical in shape from the constriction 20 to the outlet end 18. The inlet end 16 of the tube is substantially semicircular in shape, with a flat edge resting on the slide 10.

The constriction 20 comprises a convex protuberance 22 within and encircling the conduit. This defines a tapering upstream zone 24, a tapering downstream zone 26 and a short central zone 28 which is substantially cylindrical.

Fig. 2 shows the end of a micro-pipette 30 inserted into the distal end of the conduit and with its mouth positioned adjacent to the downstream zone 26.

Preferably, the tube 14 and the micro-pipette 30 are made of glass, whereas the microscope slide 10 can be made of glass or plastics material.

In order to perform the sperm preparation procedure, the tubular conduit 12 is first placed on the slide 10. Approximately 3µl of semen is placed, using a needle, into the tapering upstream zone 24 of the constriction 20. The upstream end of the tube 14 is then filled with a warm fluid medium, through the inlet end 16. This fluid medium will flow through the constriction 20 towards the distal end 18 as a capillary flow. The semen is carried through the constriction by the flow of fluid. After for example 2 minutes, if the tapering downstream zone 26 of the constriction is observed under an inverted microscope, it will be seen that there is an accumulation of sperm in the tapering downstream zone 26. These are motile sperm which have passed through the constriction and which are swimming against the flow of the medium, maintaining their position within the downstream zone of the constriction. It will be seen that the number of these motile sperm gradually increases with time. The seminal plasma, non-motile and sluggish sperm, other cellular components and bacteria pass on from zone 26 and out through the distal end 18 of the tube. The active, motile sperm swim against the flow, and form a population at the downstream zone 26.

Referring now to Fig. 3, a thin plate 32 is shown, preferably of plastics material, in the upper surface 34 of which there is formed a recess indicated generally at 36. This recess comprises a well 38 which at one end leads into an elongate groove 40. Between the well 38 and the groove 40 is inserted a piece of capillary tube 42. The piece of capillary tube 42 is shaped internally like an hourglass, in order to provide a constriction 44 to the flow of fluid therethrough. The piece of capillary tube 42 can be composed of an upstream conical segment and a downstream conical segment joined by a short intermediate segment. This means that one has a tapering upstream zone, a tapering downstream zone and a short central zone which is substantially cylindrical.

Fig. 4 shows the end of a micropipette 46 inserted into the distal end of the capillary tube 42 and with its mouth positioned in the downstream zone.

The plate 32 and the capillary tube 42 can be made of glass or plastics material, although it is preferred that the plate is made of plastics material and the capillary tube of glass.

In one practical example, approximately 3µl of semen was placed, using a needle, into the upstream conical segment of the capillary tube 42. The well 38 was then filled with 50µl of warm Ham's F-10 medium. The medium entered the capillary tube 42 and flowed through the hourglass constriction 44. After 2 minutes the tapering downstream segment was observed under an inverted microscope, using a high power field. It was observed that good numbers of sperm had accumulated inside the downstream conical segment, i.e the harvesting zone. They were swimming against the flow of medium and their numbers gradually increased with time. Using the micropipette 46, this population of sperm was aspirated and evaluated.

In both embodiments, the velocity of the fluid flowing through the narrowest portion of the constriction can be changed by changing the dimensions of the narrowest portion of the constriction. Increasing the diameter of this constriction will reduce the velocity, and vice versa. It is arranged that the dimensions of the constriction, and the viscosity of the fluid medium, are such that there is a velocity of flow of the medium through the constriction which enables motile sperm just to keep pace with the medium which exits the waist of the hourglass-shaped constriction. Using the micro-pipette, this population of sperm can be aspirated. In this way it is easy to recover an adequate number of motile, morphologically normal sperm suitable for ICSI.

With the present invention, only the most qualified sperm are selected, and the device only permits sperm that are capable of moving faster than the flow of fluid to reach the harvesting zone at the distal end of the tube. The seminal plasma and non-motile sperm are flushed from the tube.

## Claims

1. A sperm separation device, comprising a tubular conduit defining an upstream zone for receiving semen and a downstream zone from which sperm are arranged to be harvested, the upstream and downstream zones being separated by constriction means within the conduit, the constriction means being arranged to establish capillary flow of a fluid medium from the upstream zone to the downstream zone, whereby motile sperm in semen inserted into the conduit upstream from the constriction means and subjected to the flow of the fluid medium are enabled, having passed through the constriction means, to maintain a position in the downstream zone for harvesting.

2. A device according to claim 1, wherein the conduit has an upstream end of relatively large cross-section, and a downstream end of relatively small cross-section.

3. A device according to claim 1 or 2, wherein the constriction means defines a substantially hour-glass-shaped passageway within the conduit.

4. A device according to any preceding claim, wherein the constriction means is provided adjacent to the downstream end of the conduit.

5. A device according to any preceding claim, wherein the conduit is of plastics material.

6. A device according to any of claims 1 to 4, wherein the conduit is made of glass.

7. A device according to any preceding claim, wherein the conduit is supported on a baseplate.

8. A device according to claim 7, wherein the upstream zone and the downstream zone are defined by recesses in the baseplate.

9. A device according to claim 8, wherein the upstream zone is defined by a well and the downstream zone is defined by an elongate groove.

10. A device according to any preceding claim, wherein the conduit has a substantially cylindrical cross-section.

11. A device according to any of claims 1 to 9, wherein the conduit has a substantially semi-circular cross-section.

12. A device apparatus according to any preceding claim, wherein the upstream zone of the conduit is substantially funnel-shaped from the upstream end of the conduit to the constriction means.

13. A method of separating sperm, which comprises inserting sperm-containing semen into a tubular conduit upstream from constriction means within the conduit which is arranged to create capillaty flow therethrough, filling the conduit upstream from the constriction means with fluid, thereby to cause flow of semen and fluid through the constriction means, and harvesting motile sperm from the downstream side of the constriction means.

14. A method according to claim 13, in which the population of motile sperm is aspirated from the conduit using a pipette.

15. A kit of parts comprising a sperm separation device as claimed in any of claims 1 to 12 and a pipette for harvesting motile sperm.

## Patentansprüche

1. Spermientrennvorrichtung, umfassend eine röhrenförmigen Kanal, der eine stromaufwärtige Zone zur Aufnahme von Samen und eine stromabwärtige Zone, aus der Spermien geerntet werden sollen, begrenzt, wobei die stromaufwärtige und die stromabwärtige Zone durch eine Verengungseinrichtung innerhalb des Kanals getrennt sind, wobei die Verengungseinrichtung angeordnet ist, um eine Kapillarströmung eines Fluidmediums aus der stromaufwärtigen Zone zur stromabwärtigen Zone herzustellen, wodurch es bewegungsfähigen Spermien in Samen, der stromaufwärts von der Verengungseinrichtung in den Kanal eingeführt und der Strömung des Fluidmediums ausgesetzt wird, ermöglicht wird, nachdem sie durch die Verengungseinrichtung hindurchgetreten sind, eine Position zum Ernten in der stromabwärtigen Zone beizubehalten.

2. Vorrichtung nach Anspruch 1, bei welcher der Kanal ein stromaufwärtiges Ende von verhältnismäßig großem Querschnitt und ein stromabwärtiges Ende von verhältnismäßig kleinem Querschnitt aufweist.

3. Vorrichtung nach Anspruch 1 oder 2, bei welcher die Verengungseinrichtung einen im Wesentlichen sanduhrförmigen Durchlass innerhalb des Kanals begrenzt.

4. Vorrichtung nach einem vorangehenden Anspruch, bei welcher die Verengungseinrichtung benachbart zum stromabwärtigen Ende des Kanals vorgesehen ist.

5. Vorrichtung nach einem vorangehenden Anspruch, bei welcher der Kanal aus Kunststoffmaterial ist.

6. Vorrichtung nach einem der Ansprüche 1 bis 4, bei welcher der Kanal aus Glas ist.

7. Vorrichtung nach einem vorangehenden Anspruch, bei welcher der Kanal von einer Grundplatte getragen wird.

8. Vorrichtung nach Anspruch 7, bei welcher die stromaufwärtige Zone und die stromabwärtige Zone von Vertiefungen in der Grundplatte gebildet werden.

9. Vorrichtung nach Anspruch 8, bei welcher die stromaufwärtige Zone von einer Senke gebildet wird, und die stromabwärtige Zone von einer langgestreckten Nut gebildet wird.

10. Vorrichtung nach einem vorangehenden Anspruch, bei welcher der Kanal einen im Wesentlichen zylindrischen Querschnitt aufweist.

11. Vorrichtung nach einem der Ansprüche 1 bis 9, bei welcher der Kanal einen im Wesentlichen halbkreisförmigen Querschnitt aufweist.

12. Vorrichtung nach einem vorangehenden Anspruch, bei welcher die stromaufwärtige Zone des Kanals vom stromaufwärtigen Ende des Kanals bis zur Verengungseinrichtung im Wesentlichen trichterförmig ist.

13. Verfahren zum Trennen von Spermien, welches umfasst: Einführen von Spermien enthaltendem Samen in einen röhrenförmigen Kanal stromaufwärts von einer Verengungseinrichtung innerhalb des Kanals, die angeordnet ist, um durch sie hindurch eine Kapillarströmung zu erzeugen, Füllen des Kanals stromaufwärts von der Verengungseinrichtung mit Fluid, um dadurch ein Hindurchströmen von Samen und Fluid durch die Verengungseinrichtung zu bewirken, und Ernten von bewegungsfähigen Spermien aus der stromabwärtigen Seite der Verengungseinrichtung.

14. Verfahren nach Anspruch 13, bei dem die Population von bewegungsfähigen Spermien unter Verwendung einer Pipette aus dem Kanal gesaugt wird.

15. Teilesatz, umfassend eine Spermientrennvorrichtung nach einem der Ansprüche 1 bis 12 und eine Pipette zum Ernten von bewegungsfähigen Spermien.

## Revendications

1. Dispositif de sélection de spermatozoïdes, comprenant un conduit tubulaire délimitant une zone en amont pour recevoir le liquide séminal et une zone en aval au niveau de laquelle les spermatozoïdes sont disposés pour être récoltés ; les zones en amont et en aval étant séparées par des moyens d'étranglement à l'intérieur du conduit ; les moyens d'étranglement étant agencés pour générer l'écoulement capillaire d'un milieu fluide de la zone en amont vers la zone en aval, moyennant quoi les spermatozoïdes motiles, qui sont présents dans le liquide séminal, introduits dans le conduit en amont au niveau des moyens d'étranglement et soumis à l'écoulement du milieu fluide, peuvent, après passage à travers les moyens d'étranglement, maintenir une position dans la zone en aval destinée à la récolte.

2. Dispositif selon la revendication 1 dans lequel le conduit est doté d'une extrémité en amont de coupe transversale relativement large, et d'une extrémité en aval de coupe transversale relativement petite.

3. Dispositif selon la revendication 1 ou 2 dans lequel les moyens d'étranglement définissent un passage sensiblement en forme de sablier à l'intérieur du conduit.

4. Dispositif selon l'une quelconque des revendications précédentes dans lequel les moyens d'étranglement sont prévus adjacents à l'extrémité en aval du conduit.

5. Dispositif selon l'une quelconque des revendications précédentes dans lequel le conduit est en matériau plastique.

6. Dispositif selon l'une quelconque des revendications 1 à 4 dans lequel le conduit est en verre.

7. Dispositif selon l'une quelconque des revendications précédentes dans lequel le conduit est supporté par une plaque base.

8. Dispositif selon la revendication 7 dans lequel la zone en amont et la zone en aval sont délimitées par des creux dans la plaque base.

9. Dispositif selon la revendication 8 dans lequel la zone en amont est délimitée par un évidement et la zone en aval est délimitée par une rainure longitudinale.

10. Dispositif selon l'une quelconque des revendications précédentes dans lequel le conduit présente une coupe transversale sensiblement cylindrique.

11. Dispositif selon l'une quelconque des revendications 1 à 9 dans lequel le conduit présente une coupe transversale sensiblement semi-circulaire.

12. Appareil de dispositif selon l'une quelconque des revendications précédentes dans lequel la zone en amont du conduit présente une forme sensiblement en entonnoir à partir de l'extrémité en amont du conduit jusqu'aux moyens d'étranglement.

13. Procédé de sélection de spermatozoïdes comprenant l'introduction de liquide séminal contenant des spermatozoïdes dans un conduit tubulaire en amont au niveau de moyens d'étranglement situés à l'intérieur du conduit qui sont agencés pour générer un écoulement capillaire à travers celui-ci, le remplissage du conduit en amont au niveau des moyens d'étranglement avec du fluide, donnant ainsi lieu à un écoulement de liquide séminal et de fluide le long des moyens d'étranglement, et la récolte des spermatozoïdes motiles en aval des moyens d'étranglement.

14. Procédé selon la revendication 13 dans lequel la population de spermatozoïdes motiles est aspirée à partir du conduit en utilisant une pipette.

15. Ensemble d'éléments comprenant un dispositif de sélection de spermatozoïdes selon l'une quelconque des revendications 1 à 12 et une pipette pour la récolte des spermatozoïdes motiles.
